# EUROPÄISCHE PATENTSCHRIFT

(11) **EP 1 374 859 B1**
(45) Veröffentlichungstag und Bekanntmachung des Hinweises auf die Patenterteilung: **07.06.2006**
(21) Anmeldenummer: 03014715.1
(22) Anmeldetag: 27.06.2003
(51) Int. Cl.: A61K 9/22, A61K 31/216, A61K 31/485

(54) **Feste pharmazeutische Zusammensetzung enthaltend Tilidinhydrochlorid**
Solid pharmaceutical composition comprising tilidine hydrochloride
Composition pharmaceutique solide du chlorhydrate de tilidine

(30) Priorität: 28.06.2002 DE 10229216
(43) Veröffentlichungstag der Anmeldung: 02.01.2004
(73) Patentinhaber: STADA ARZNEIMITTEL AG, 61118 Bad Vilbel (DE)
(72) Erfinder: Schumann, Christof, 35767 Breitscheid-Erdbach (DE); Renz, Jessica, 61231 Bad Nauheim (DE)
(74) Vertreter: Hamm, Volker

(56) Entgegenhaltungen:
- EP-A- 0 960 619
- WO-A-94/10129
- DE-A- 2 530 563
- DE-A- 2 605 243

## Beschreibung

Die Erfindung betrifft stabile, feste pharmazeutische Zusammensetzungen umfassend Tilidinhydrochlorid als Wirkstoff.

Tilidin, dessen chemische Bezeichnung [(±)-Ethyl-(trans-2-dimethylamino-1-phenyl-3-cyclohexen-trans-1-carboxylat)] lautet, ist ein entfernt mit Morphin verwandtes Opioidanalgetikum, das enteral rasch resorbiert wird und sich zur Behandlung sehr starker Schmerzen besonders eignet. Das Hydrochlorid-Salz des Tilidins, auch in Form seines Hemihydrates, wird seit langem in Form von Lösungen unter der Handelsbezeichnung "Valoron N" vertrieben. Da Tilidin und seine Salze Suchtgifte darstellen, werden Tilidin enthaltende Arzneimittel zur Verhinderung des Missbrauches üblicherweise mit Morphin-Antagonisten kombiniert, vorzugsweise mit Naloxon.

In der Fachwelt bestand und besteht das Vorurteil, dass Tilidinhydrochlorid sowie dessen Hydrate sich nicht zu festen Zubereitungen verarbeiten lassen, da sie in solchen festen Zubereitungen instabil sind. So wird in WO 94/10129 ausgeführt, dass Verreibungen von Tilidinhydrochlorid-Hemihydrat mit üblicherweise pharmazeutisch eingesetzten Hilfsstoffen bereits nach 28 Tagen Lagerung bei 60°C Verfärbungen zeigten. Bei der Verarbeitung von Tilidinhydrochlorid-Hemihydrat mit Naloxonhydrochlorid-Dihydrat, hydriertem Rizinusöl, Lactose, Hydroxyethylcellulose, Stearinsäure, Tablettose und Magnesiumstearat zu Schmelzgranulat-Tabletten zeigten die Tabletten bereits nach 2 Tagen Lagerung in Braunglas bei 22°C orange-graue Verfärbungen. Zur Umgehung der als nicht lösbar angesehenen Stabilitätsprobleme in Verbindung mit Tilidinhydrochlorid wurde gemäß WO 94/10129 die Verwendung von Tilidinorthophosphat anstelle des Hydrochlorids vorgeschlagen. Tilidinhydrogenorthophosphat ist in den derzeit in Deutschland vertriebenen festen Arzneiformen umfassend Tilidin enthalten.

Auch in WO 00/38656 wird berichtet, dass sich Tilidinhydrochlorid nicht zu festen Darreichungsformen verarbeiten lässt, da es sich in Verbindung mit festen Hilfsstoffen sehr schnell bei der Herstellung und bei der Lagerung zersetzt, was sich auch durch Verfärbungen bemerkbar macht. Zur Überwindung dieser Stabilitätsprobleme wird in WO 00/38656 vorgeschlagen, Tilidinmesylat anstelle des Hydrochlorids zu verwenden.

Des weiteren wird in EP 0 960 619 A diskutiert, dass die in Zusammenhang mit einigen Tilidinsalzen und insbesondere mit Tilidinhydrochlorid im Stand der Technik diskutierten Stabilitätsprobleme dadurch überwunden werden können, indem in festen Zusammensetzungen Tilidin in Form eines Salzes eines nicht toxischen Komplexbildners für 2- oder 3-wertige Metallkationen oder in Kombination mit solchen Komplexbildnern als Stabilisatoren verwendet wird. Als geeignete Stabilisatoren werden Citronensäure und Weinsäure als besonders bevorzugt genannt.

Tilidin wird in Form seines Hydrochlorid-Hemihydrats seit langem in flüssigen Arzneiformen, beispielsweise in Form von Lösungen vertrieben. Es wäre daher wünschenswert, auch über feste, oral zu verabreichende Arzneiformen zu verfügen, die das seit langem verwendete Tilidinhydrochlorid enthalten, da irgendwelche Überempfindlichkeitsreaktionen von Patienten, die zuvor flüssige Tilidinhydrochlorid-Präparate eingenommen haben, bei der Umstellung dieser Patienten auf feste Arzneiformen von vornherein ausgeschlossen werden können. Der Vorteil fester Arzneiformen liegt insbesondere darin, dass sie eine steuerbare, vorzugsweise verzögerte (retardierte) Freisetzung des Tilidins aus der Arzneiform ermöglichen, so dass eine gleichmäßige Schmerzbehandlung über einen längeren Zeitraum möglich wird. Dies ist insbesondere bei der Bekämpfung chronischer Schmerzen vorteilhaft.

Im Rahmen der der vorliegenden Erfindung zugrundeliegenden Untersuchungen wurde überraschend gefunden, dass sich entgegen dem auf dem Fachgebiet vorherrschenden Vorurteil Tilidinhydrochlorid, ggf. in Kombination mit Naloxon, unter Verwendung üblicher pharmazeutischer Hilfsstoffe und ggf. Retardierungsmittel problemlos zu festen, oral zu verabreichenden pharmazeutischen Zusammensetzungen formulieren lässt, die über lange Zeiträume stabil sind, d.h. keinerlei Zersetzungsreaktionen unterliegen oder Verfärbungen zeigen, und zwar auch ohne den Zusatz irgendwelcher Stabilisatoren.

Gemäß der vorliegenden Erfindung werden somit erstmals stabile, stabilisatorfreie pharmazeutische Zusammensetzungen in fester Form, vorzugsweise in Form von Tabletten, angegeben, die Tilidinhydrochlorid, ggf. in Form eines seiner Hydrate, als Wirkstoff enthalten.

Gegenstand der Erfindung ist somit eine Zusammensetzung nach Anspruch 1. Die Unteransprüche betreffen bevorzugte Ausführungsformen dieser Zusammensetzung.

Die erfindungsgemäßen Zusammensetzungen sind vorzugsweise so formuliert, dass das Tilidinhydrochlorid kontrolliert aus den Zusammensetzungen freigesetzt wird. Wie allgemein bekannt ist, lässt sich durch spezifische Auswahl geeigneter Retardierungsmittel und deren Mengen der Zeitraum, über den die Wirkstoffabgabe erfolgt, steuern. Als Retardierungsmittel eignen sich Methylcellulose, Ethylcellulose, Carboxymethylcellulose, Hydroxymethylcellulose, Hydroxypropylcellulose, Hydroxypropylmethylcellulose oder Hydroxyethylcellulose, Polyvinylalkohol, Polyvinylpyrrolidon, Alginate, Acrylsäurecopolymere, Methylacrylsäurecopolymere, Fettalkohole wie beispielsweise Cetostearylalkohol, Glycerinester von Fettsäuren sowie Wachse wie Bienenwachse oder Paraffinwachse. Besonders bevorzugte Retardierungsmittel sind Hydroxypropylmethylcellulose, Acrylharze, Polyvinylpyrrolidon, Cetostearylalkohol sowie Celluloseether. Es können ein oder mehrere geeignete Retardierungsmittel in den erfindungsgemäßen Zusammensetzungen enthalten sein. Dabei können die Freisetzungseigenschaften durch Auswahl und Menge des Retardierungsmittel eingestellt werden; im Falle der Polymeren auf Cellulosebasis kann eine Beeinflussung der Freisetzungsgeschwindigkeit in an sich bekannter Weise, wie z.B. aus EP 0 068 446 A bekannt, auch durch die Auswahl der Viskosität des Polymers erfolgen. Gemäß besonders bevorzugter Ausführungsformen der Erfindung wird Hydroxypropylmethylcellulose mit Viskositäten von 100 bzw. 4000 mPa.s ggf. im Gemisch, verwendet.

Die Gesamtmenge an zugesetztem Retardierungsmittel in den Zusammensetzungen hängt von den gewünschten Freisetzungseigenschaften der Zusammensetzung ab, liegt im allgemeinen jedoch zwischen 1 und 90 Gew.-%, bevorzugt zwischen 15 und 65 Gew.-%, bezogen auf die Gesamtzusammensetzung.

Die erfindungsgemäßen Zusammensetzungen werden vorzugsweise so formuliert, dass die Freisetzung des Wirkstoffes über einen Zeitraum von 8 bis 12 Stunden, vorzugsweise über 24 Stunden erfolgt, so dass über die genannten Zeiträume eine gleichmäßige Konzentration an Tilidin bzw. seiner physiologisch wirksamen Metaboliten im Blutplasma gegeben ist.

Die *in vitro*-Freisetzungsrate von Tilidinhydrochlorid aus den erfindungsgemäßen Zusammensetzungen beträgt vorzugsweise 20 bis 40 Gew.-% nach einer Stunde, 30 bis 60 Gew.-% nach zwei Stunden, 50 bis 80 Gew.-% nach vier Stunden, 70 bis 90 Gew.-% nach sechs Stunden, 80 bis 90 Gew.-% nach acht Stunden und mehr als 90 Gew.-% nach 10 Stunden.

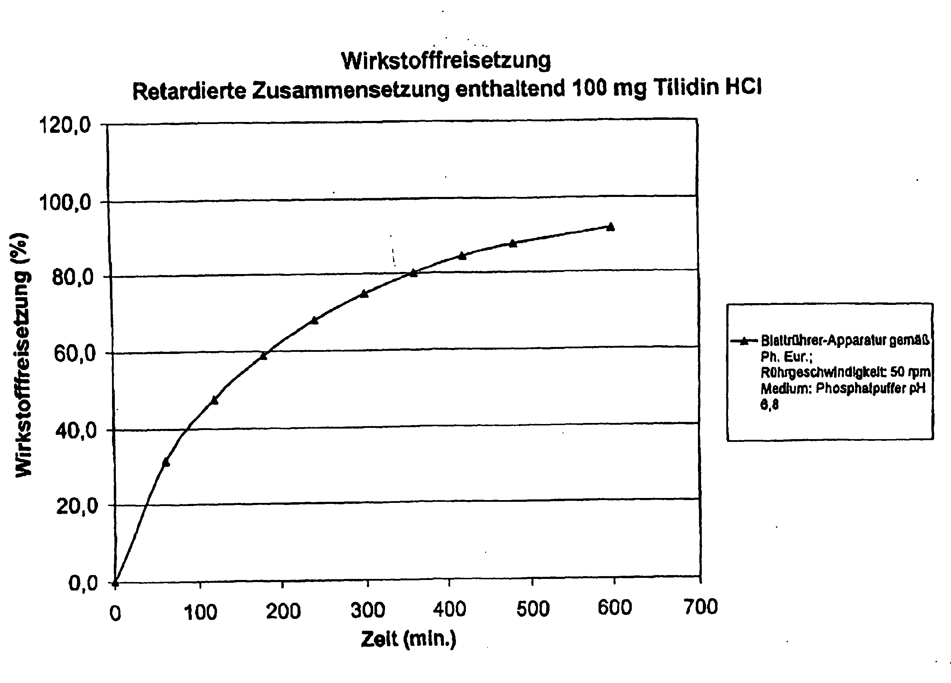

In Figur 1 ist ein Freisetzungsprofil für eine erfindungsgemäße Zusammensetzung exemplifiziert.

Die Zusammensetzungen gemäß der Erfindung enthalten neben Tilidinhydrochlorid bevorzugt Naloxon, das mit einer vergleichbaren Geschwindigkeit aus der Zusammensetzung freigesetzt wird, wie das Tilidinhydrochlorid.

Neben Tilidinhydrochlorid, ggf Naloxon und dem Retardierungsmittel enthalten die erfindungsgemäßen Zusammensetzungen übliche pharmazeutische Hilfsstoffe wie Bindemittel, Füllstoffe, Weichmacher, Sprengmittel, Schmiermittel, Konservierungsmittel, Filmüberzugsmittel und dergleichen. Die Herstellung der endgültigen Arzneiform kann mittels üblicher Verfahren, z.B. durch Feuchtgranulation, Trockengranulation oder Direktverpressung erfolgen. Die bevorzugten Arzneiformen sind Tabletten oder in Gelatinekapseln gefüllte Pellets oder Granulat.

Der Gehalt an Tilidinhydrochlorid in den erfindungsgemäßen Zusammensetzungen beträgt vorzugsweise 10 bis 500 mg, besonders bevorzugt 50 bis 200 mg, wobei Tabletten umfassend 50, 100, 150 und 200 mg Tilidinhydrochlorid besonders bevorzugt sind.

Sofern enthalten, beträgt der Gehalt an Naloxon in den erfindungsgemäßen Zusammensetzungen vorzugsweise 5 bis 15 Gew.-%, bezogen auf die Menge an enthaltenem Tilidinhydrochlorid.

Die nachfolgenden Beispiele veranschaulichen die Erfindung.

### Beispiel 1

| Inhaltsstoffe | mg/Tablette |
|---|---|
| 1. Tilidinhydrochlorid x 0,5H₂O | 102,9 mg |
| 2. Naloxonhydrochlorid x 2H₂O | 8,8 mg |
| 3. Hydroxypropylmethylcellulose, Viskosität 4000 mPa·s | 55 mg |
| 4. Hydroxypropylmethylcellulose, Viskosität 100 eR mPa·s | 35 mg |
| 5. Mikrokristalline Cellulose | 149 mg |
| 6. Hochdisperses Siliciumdioxid | 3 mg |
| 7. Magnesiumstearat | 2 mg |

Tilidinhydrochlorid-Hemihydrat, Naloxonhydrochlorid-Dihydrat, Hydroxypropylmethylcellulose mit einer Viskosität von 4000 mPa·s, Hydroxypropylmethylcellulose mit einer Viskosität von 100 mPa·s mikrokristalline Cellulose und hochdisperses Siliciumdioxid werden durch ein Sieb (Maschenweite 1000 µm) gesiebt und vermischt. Gesiebtes Magnesiumstearat wird zur Pulvermischung hinzugegeben und es wird erneut vermischt. Anschließend wird die Pulvermischung direkt zu Tabletten verpresst. Die so hergestellten Tablettenkerne werden mit einem Opadry®-Überzug versehen.

### Beispiel 2

| Inhaltsstoffe | mg/Tablette |
|---|---|
| 1. Tilidinhydrochlorid x 0,5H₂O | 102,9 mg |
| 2. Naloxonhydrochlorid x 2H₂O | 8,8 mg |
| 3. Hydroxypropylmethylcellulose, Viskosität 4000 mPa·s | 75 mg |
| 4. Mikrokristalline Cellulose | 149 mg |
| 5. Hochdisperses Siliciumdioxid | 3 mg |
| 6. Magnesiumstearat | 2 mg |

Wie im vorhergehenden Beispiel werden die Komponenten 1 bis 5 gesiebt und vermischt. Magnesiumstearat wird gesiebt und zur Pulvermischung hinzugegeben, wonach erneut gemischt wird. Die Pulvermischung wird direkt zu Tabletten verpresst.

### Beispiel 3

| Inhaltsstoffe | mg/Tablette |
|---|---|
| 1. Tilidinhydrochlorid x 0,5H₂O | 102,9 mg |
| 2. Naloxonhydrochlorid x 2H₂O | 8,8 mg |
| 3. Polyvinylpyrrolidon | 10 mg |
| 4. Polymethacrylat | 37,5 mg |
| 5. Cetostearylalkohol | 140 mg |
| 6. Magnesiumstearat | 3 mg |
| 7. Aceton/Isopropanol (1:1) | q.s. |

Die Komponenten 1.-3. werden gemischt. Zu dieser Pulvermischung wird unter weiterem Mischen eine Lösung von Polymethacrylat in einer 1:1 Mischung von Aceton und Isopropanol gegeben; diese Lösung dient als Granulierflüssigkeit. Das entstandene Granulat wird getrocknet und gesiebt. Geschmolzener Cetostearylalkohol wird zu dem noch warmen Granulat gegeben und untergemischt. Nach der Abkühlung wird die erhaltene Granulatmischung gesiebt, zu der gesiebten Granulatmischung werden Talkum und Magnesiumstearat gegeben und untergemischt. Die so gebildete Granulatmischung wird dann zu Tabletten verpresst.

### Beispiel 4

| Inhaltsstoffe | mg/Tablette |
|---|---|
| 1. Tilidinhydrochlorid x 0,5H₂O | 102,9 mg |
| 2. Naloxonhydrochlorid x 2H₂O | 8,8 mg |
| 3. Gemisch aus Lactose und mikrokristalliner Cellulose | 170 mg |
| 4. Polyvinylpyrrolidon | 25 mg |
| 5. Celluloseether | 35 mg |
| 6. Talkum | 6 mg |
| 7. Hochdisperses Siliciumdioxid | 2 mg |
| 8. Magnesiumstearat | 2 mg |

Die Komponenten 1.-7. werden gesiebt und gemischt. Zu der Pulvermischung wird Magnesiumstearat gegeben und untergemischt. Die erhaltene Mischung wird zu Tabletten verpresst, die mit einem Opadry®-Film überzogen werden.

### Beispiel 5

| Inhaltsstoffe | mg/Tablette |
|---|---|
| 1. Tilidinhydrochlorid x 0,5H₂O | 102,9 mg |
| 2. Naloxonhydrochlorid x 2H₂O | 8,8 mg |
| 3. Hydroxypropylmethylcellulose, Viskosität 4000 mPa·s | 75 mg |
| 4. Polyvinylpyrrolidon | 21 mg |
| 5. Mikrokristalline Cellulose | 170 mg |
| 6. Hochdisperses Siliciumdioxid | 3 mg |
| 7. Magnesiumstearat | 2 mg |
| 8. Wasser, gereinigt (wird wieder entfernt) | 74 mg |

Die Komponenten 1.-3. werden gesiebt und gemischt. Zu dieser Pulvermischung wird unter Mischen eine Lösung von Polyvinylpyrrolidon in Wasser, die als Granulierflüssigkeit dient, gegeben. Das erhaltene Granulat wird getrocknet und gesiebt. Mikrokristalline Cellulose und hochdisperses Siliciumdioxid werden gesiebt, dem erhaltenen Granulat hinzugegeben und untergemischt. Danach wird gesiebtes Magnesiumstearat zugegeben und erneut gemischt. Die erhaltene Granulatmischung wird dann zu Tabletten verpresst.

### Beispiel 6

Die Zusammensetzung gemäß Beispiel 1 wurde unter Stressbedingungen bei 40°C und 75 % relativer Luftfeuchtigkeit 4, 8 oder 12 Wochen sowie 6 Monate gelagert. Nach der angegebenen Lagerzeit zeigte die Zusammensetzung keinerlei Verfärbungen. Mittels HPLC-Untersuchungen wurde auf Verunreinigungen in den Zusammensetzungen vor und nach der Lagerung untersucht. Zum Vergleich wurden identische Untersuchungen mit dem im Handel befindlichen Produkt umfassend Tilidinorthophosphat als Wirkstoff durchgeführt. Die erhaltenen Ergebnisse sind in den nachfolgenden Tabellen 1-4 zusammengefasst.

### Tabellen 1-4:

Verunreinigungen in den erfindungsgemäßen Zusammensetzungen und dem im Handel befindlichen Präparat vor und nach Lagerung unter Stressbedingungen

**Tabelle 1:**

| **Gelagert bei: 40°C / 75 % r.h.** | **4 Wochen / Reinheit** |
|---|---|
| Zusammensetzung | Bekannte Verunreinigungen: |
| gemäß Beispiel 1 | *0,07* % *(0,03 % Noroxymorphone; 0,04 % Nor-Tilidin)* |
| | unbekannte Verunreinigungen, einzeln: |
| | *0,054* % |
| | unbekannte Verunreinigungen, gesamt: |
| | 0,14 % |
| | Gesamte Verunreinigungen: |
| | 0,21 % |
| Im Handel befindliche Zusammensetzung mit Tilidinorthophosphat als Wirkstoff | Bekannte Verunreinigungen: |
| | 0,25 % (0, 03 % Noroxymorphone; 0,08 % Nor-Tilidin; 0, 04 % cis-Tilidin; 0,10 % Bisnaloxon) |
| | unbekannte Verunreinigungen, einzeln: |
| | *0,068* % |
| | unbekannte Verunreinigungen, gesamt: |
| | 0,13% |
| | Gesamte Verunreinigungen: |
| | *0,38%* |

**Tabelle 2:**

| **Gelagert bei: 40°C / 75 % r.h.** | **8 Wochen / Reinheit** |
|---|---|
| Zusammensetzung gemäß Beispiel 1 | Bekannte Verunreinigungen: |
| | *0,04 % (0,04 % Nor-Tilidin)* |
| | unbekannte Verunreinigungen, einzeln: |
| | *0,052 %* |
| | unbekannte Verunreinigungen, gesamt: |
| | *0,14%* |
| | Gesamte Verunreinigungen: |
| | 0,18% |
| Im Handel befindliche Zusammensetzung mit Tilidinorthophosphat als Wirkstoff | Bekannte Verunreinigungen: |
| | *0,32* % *(0,07 % Noroxymorphone; 0,08 % Nor-Tilidin; 0,03 % cis-Tilidin; 0,14 % Bisnaloxon)* |
| | unbekannte Verunreinigungen, einzeln: |
| | *0,082* % |
| | unbekannte Verunreinigungen, gesamt: |
| | 0,13% |
| | Gesamte Verunreinigungen: |
| | *0,45 %* |

**Tabelle 3:**

| **Gelagert bei: 40°C / 75 % r.h.** | **12 Wochen / Reinheit** |
|---|---|
| Zusammensetzung gemäß Beispiel 1 | Bekannte Verunreinigungen: |
| | *0,07* % *(0,07 % Nor-Tilidin)* |
| | unbekannte Verunreinigungen, einzeln: |
| | *0,052* % |
| | unbekannte Verunreinigungen, gesamt: |
| | *0,16%* |
| | Gesamte Verunreinigungen: |
| | *0,23 %* |
| Im Handel befindliche Zusammensetzung mit Tilidinorthophosphat als Wirkstoff | Bekannte Verunreinigungen: |
| | *0,41 % (0,09 % Noroxymorphone; 0,13 % Nor-Tilidin; 0,04 % cis-Tilidin; 0,11 % Bisnaloxon; 0,04 % Michael Addukt)* |
| | unbekannte Verunreinigungen, einzeln: |
| | *0,061 %* |
| | unbekannte Verunreinigungen, gesamt: |
| | *0,15%* |
| | Gesamte Verunreinigungen: |
| | *0,56 %* |

**Tabelle 4:**

| **Gelagert bei: 40°C / 75 % r.h.** | **6 Monate / Reinheit** |
|---|---|
| Zusammensetzung gemäß Beispiel 1 | Bekannte Verunreinigungen: |
| | *0,10* % *(0,10 % Nor-Tilidin)* |
| | unbekannte Verunreinigungen, einzeln: |
| | *0,053* % |
| | unbekannte Verunreinigungen, gesamt: |
| | *0,15%* |
| | Gesamte Verunreinigungen: |
| | *0,25 %* |
| Im Handel befindliche Zusammensetzung mit Tilidinorthophosphat als Wirkstoff | Bekannte Verunreinigungen: |
| | *0,43 % (0,11 % Noroxymorphone; 0,15 % Nor-Tilidin; 0,06 % cis-Tilidin; 0,11 % Bisnaloxon)* |
| | unbekannte Verunreinigungen, einzeln: |
| | *0, 096 %* |
| | unbekannte Verunreinigungen, gesamt: |
| | *0,17 %* |
| | Gesamte Verunreinigungen: |
| | 0,60 % |

Wie aus den Tabellen ersichtlich wird, nahmen die Verunreinigungen in den erfindungsgemäßen Zusammensetzungen nach der Lagerung unter Stressbedingungen nur unwesentlich zu, wobei die Gesamtmenge an Verunreinigungen sogar unter der des momentan im Handel befindlichen Präparats liegt.

## Patentansprüche

1. Feste, stabile pharmazeutische Zusammensetzung für die orale Verabreichung umfassend Tilidinhydrochlorid bzw. eines seiner Hydrate, einen Morphin-Antagonist sowie pharmazeutische Hilfsstoffe, mit der Maßgabe, dass solche Zusammensetzungen ausgenommen sind, die Komplexbildner für 2- oder 3-wertige Metallkationen oder Pyrazolessigsäure enthalten
**dadurch gekennzeichnet, dass** sie mindestens ein Retardierungsmittel, ausgewählt aus Alkylcellulosen, Alginate, Acrylsäurecopolymere, Methylacrylsäurecopolymere, Hydroxyalkylcellulosen, Polyvinylpyrrolidon, Fettalkoholen, Polyvinylalkoholen, Fettsäure-Glycerinestem und Wachsen, enthält.

2. Zusammensetzung nach Anspruch 1,
**dadurch gekennzeichnet, dass** die Menge an Tilidinhydrochlorid oder dessen Hydrat 10 bis 500 mg, vorzugsweise 50 bis 200 mg beträgt.

3. Zusammensetzung nach einem der Ansprüche 1 oder 2,
**dadurch gekennzeichnet, dass** der Morphin-Antagonist, Naloxon bzw. dessen pharmazeutisch annehmbares Salz bzw. Hydrat ist.

4. Zusammensetzung gemäß einem der vorstehenden Ansprüche in der Form von Tabletten, Granulaten, Pulvern oder Pellets.

## Claims

1. A solid, stable pharmaceutical composition for oral administration comprising tilidine hydrochloride or one of the hydrates thereof, a morphine antagonist together with pharmaceutical auxiliary substances, with the proviso that those compositions containing complexing agents for di- or trivalent metal cations or pyrazole acetic acid are excluded,
**characterised in that** said composition contains at least one controlled-release agent selected from among alkylcelluloses, alginates, acrylic acid copolymers, methylacrylic acid copolymers, hydroxyalkylcelluloses, polyvinylpyrrolidone, fatty alcohols, polyvinyl alcohols, fatty acid glycerol esters and waxes.

2. A composition according to claim 1,
**characterised in that** the quantity of tilidine hydrochloride or the hydrate thereof amounts to 10 to 500 mg, preferably to 50 to 200 mg.

3. A composition according to either of claim 1 or claim 2,
**characterised in that** the morphine antagonist is naloxone or the pharmaceutically acceptable salt or hydrate thereof.

4. A composition according to any one of the preceding claims in the form of tablets, granules, powders or pellets.

## Revendications

1. Composition pharmaceutique stable solide, destinée à l'administration orale comprenant le chlorhydrate de tilidine ou l'un de ses hydrates, un antagoniste de morphine, ainsi que des adjuvants pharmaceutiques, à condition que les compositions qui contiennent des complexants pour des cations métalliques bivalents ou trivalents ou de l'acide pyrazole-acétique, soient exclues,
**caractérisée en ce qu'**elle contient un agent retardateur choisi parmi les alkylcelluloses, les alginates, les copolymères de l'acide acrylique, les copolymères de l'acide méthacrylique, les hydroxyalkylcelluloses, la polyvinylpyrrolidone, les alcools gras, les poly(alcool vinylique)s, les esters de glycérine avec un acide gras et les cires.

2. Composition selon la revendication 1,
**caractérisée en ce que** la quantité de chlorhydrate de tilidine ou de son hydrate, est de 10 à 500 mg, de préférence de 50 à 200 mg.

3. Composition selon l'une quelconque des revendications 1 ou 2,
**caractérisée en ce que** l'antagoniste de morphine est le Naloxon ou ses sels ou hydrates, acceptables sur le plan pharmaceutique.

4. Composition selon l'une quelconque des revendications précédentes, sous forme de comprimés, granulés, poudres ou pellets.
